# EUROPEAN PATENT APPLICATION

(11) **EP 1 262 558 A1**
(43) Date of publication of application: **04.12.2002**
(21) Application number: 01912232.4
(22) Date of filing: 09.03.2001
(51) Int. Cl.: C12Q 1/25, C12N 15/52, G01N 33/50, G01N 33/15

(54) **METHOD OF SCREENING INDUCIBLE NITRIC OXIDE SYNTHASE ACTIVATION INHIBITOR**

(30) Priority: 10.03.2000 JP 2000072480
(71) Applicant: FUJISAWA PHARMACEUTICAL CO., LTD., Osaka-shi Osaka 541-8514 (JP)
(72) Inventor: ISHII, Yoshinori, Fujisawa Pharmaceutical Co. Ltd, Osaka-shi, Osaka541-8514 (JP); UEDA, Yoshiko, Fujisawa Pharmaceutical Co., Ltd, Osaka-shi, Osaka 541-8514 (JP); IWAMI, Morita, Fujisawa Pharmaceutical Co., Ltd, Osaka-shi, Osaka 541-8514 (JP); ARAKAWA, Hiroyuki, Fujisawa Pharmaceutical Co. Ltd, Osaka-shi, Osaka 541-8514 (JP); NOTSU, Yoshitada, Hadano-shi, Kanagawa 259-1321 (JP)
(74) Representative: Gille Hrabal Struck Neidlein Prop Roos
(86) International application number: JP0101865
(87) International publication number: WO01066791

(57) **Abstract**

Methods of screening for iNOS activity inhibitors are provided using the binding activity of candidate compounds towards the iNOS monomer (or a mutant thereof) as an index. Dimerization of iNOS is the final step in the acquisition of iNOS enzyme activity. The screening methods according to this invention enable quick screening of iNOS inhibitory compounds that have excellent specificity and rapid action properties through simple manipulations.

SEQUENCE LISTING

<110> Fujisawa Pharmaceutical Co., Ltd.

<120> SCREENING METHODS FOR INHIBITORS OF INDUCIBLE NITROGEN OXIDE
      SYNTHASE ACTIVATION

<130> F3-106PCT

<140>
<141>

<150> JP 2000-72480
<151> 2000-03-10

<160> 12

<170> PatentIn Ver. 2.0

<210> 1
<211> 3462
<212> DNA
<213> Homo sapiens

<400> 1
atggcctgtc cttggaaatt tctgttcaag accaaattcc accagtatgc aatgaatggg 60

gaaaaagaca tcaacaacaa tgtggagaaa gccccctgtg ccacctccag tccagtgaca 120

caggatgacc ttcagtatca caacctcagc aagcagcaga atgagtcccc gcagcccctc 180

gtggagacgg gaaagaagtc tccagaatct ctggtcaagc tggatgcaac cccattgtcc 240

tccccacggc atgtgaggat caaaaactgg ggcagcggga tgactttcca agacacactt 300

caccataagg ccaaagggat tttaacttgc aggtccaaat cttgcctggg gtccattatg 360

actcccaaaa gtttgaccag aggacccagg gacaagccta cccctccaga tgagcttcta 420

cctcaagcta tcgaatttgt caaccaatat tacggctcct tcaaagaggc aaaaatagag 480

gaacatctgg ccagggtgga agcggtaaca aaggagatag aaacaacagg aacctaccaa 540

ctgacgggag atgagctcat cttcgccacc aagcaggcct ggcgcaatgc cccacgctgc 600

attgggagga tccagtggtc caacctgcag gtcttcgatg cccgcagctg ttccactgcc 660

cgggaaatgt ttgaacacat ctgcagacac gtgcgttact ccaccaacaa tggcaacatc 720

aggtcggcca tcaccgtgtt cccccagcgg agtgatggca agcacgactt ccgggtgtgg 780

aatgctcagc tcatccgcta tgctggctac cagatgccag atggcagcat cagaggggac 840

cctgccaacg tggaattcac tcagctgtgc atcgacctgg gctggaagcc caagtacggc 900

cgcttcgatg tggtccccct ggtcctgcag gccaatggcc gtgaccctga gctcttcgaa 960

atcccacctg accttgtgct tgaggtggcc atggaacatc ccaaatacga gtggtttcgg 1020

gaactggagc taaagtggta cgccctgcct gcagtggcca acatgctgct tgaggtgggc 1080

ggcctggagt tcccagggtg ccccttcaat ggctggtaca tgggcacaga gatcggagtc 1140

cgggacttct gtgacgtcca gcgctacaac atcctggagg aagtgggcag gagaatgggc 1200

ctggaaacgc acaagctggc ctcgctctgg aaagaccagg ctgtcgttga gatcaacatt 1260

gctgtgctcc atagtttcca gaagcagaat gtgaccatca tggaccacca ctcggctgca 1320

gaatccttca tgaagtacat gcagaatgaa taccggtccc gtgggggctg cccggcagac 1380

tggatttggc tggtccctcc catgtctggg agcatcaccc ccgtgtttca ccaggagatg 1440

ctgaactacg tcctgtcccc tttctactac tatcaggtag aggcctggaa aacccatgtc 1500

tggcaggacg agaagcggag acccaagaga agagagattc cattgaaagt cttggtcaaa 1560

gctgtgctct ttgcctgtat gctgatgcgc aagacaatgg cgtcccgagt cagagtcacc 1620

atcctctttg cgacagagac aggaaaatca gaggcgctgg cctgggacct gggggcctta 1680
ttcagctgtg ccttcaaccc caaggttgtc tgcatggata agtacaggct gagctgcctg 1740
gaggaggaac ggctgctgtt ggtggtgacc agtacgtttg gcaatggaga ctgccctggc 1800
aatggagaga aactgaagaa atcgctcttc atgctgaaag agctcaacaa caaattcagg 1860
tacgctgtgt ttggcctcgg ctccagcatg taccctcggt tctgcgcctt tgctcatgac 1920
attgatcaga agctgtccca cctgggggcc tctcagctca ccccgatggg agaaggggat 1980
gagctcagtg ggcaggagga cgccttccgc agctgggccg tgcaaacctt caaggcagcc 2040
tgtgagacgt ttgatgtccg aggcaaacag cacattcaga tccccaagct ctacacctcc 2100
aatgtgacct gggacccgca ccactacagg ctcgtgcagg actcacagcc tttggacctc 2160
agcaaagccc tcagcagcat gcatgccaag aacgtgttca ccatgaggct caaatctcgg 2220
cagaatctac aaagtccgac atccagccgt gccaccatcc tggtggaact ctcctgtgag 2280
gatggccaag gcctgaacta cctgccgggg gagcaccttg gggtttgccc aggcaaccag 2340
ccggccctgg tccaaggtat cctggagcga gtggtggatg gccccacacc ccaccagaca 2400
gtgcgcctgg aggccctgga tgagagtggc agctactggg tcagtgacaa gaggctgccc 2460
ccctgctcac tcagccaggc cctcacctac ttcctggaca tcaccacacc cccaacccag 2520
ctgctgctcc aaaagctggc ccaggtggcc acagaagagc ctgagagaca gaggctggag 2580
gccctgtgcc agccctcaga gtacagcaag tggaagttca ccaacagccc cacattcctg 2640
gaggtgctag aggagttccc gtccctgcgg gtgtctgctg gcttcctgct ttcccagctc 2700
cccattctga agcccaggtt ctactccatc agctcctccc gggatcacac gcccacagag 2760
atccacctga ctgtggccgt ggtcacctac cacacccgag atggccaggg tcccctgcac 2820
cacggcgtct gcagcacatg gctcaacagc ctgaagcccc aagacccagt gccctgcttt 2880
gtgcggaatg ccagcggctt ccacctcccc gaggatccct cccatccttg catcctcatc 2940
gggcctggca caggcatcgc gcccttccgc agtttctggc agcaacggct ccatgactcc 3000
cagcacaagg gagtgcgggg aggccgcatg accttggtgt ttgggtgccg ccgcccagat 3060
gaggaccaca tctaccagga ggagatgctg gagatggccc agaagggggt gctgcatgcg 3120
gtgcacacag cctattcccg cctgcctggc aagcccaagg tctatgttca ggacatcctg 3180

cggcagcagc tggccagcga ggtgctccgt gtgctccaca aggagccagg ccacctctat 3240

gtttgcgggg atgtgcgcat ggcccgggac gtggcccaca ccctgaagca gctggtggct 3300

gccaagctga aattgaatga ggagcaggtc gaggactatt tctttcagct caagagccag 3360

aagcgctatc acgaagatat ctttggtgct gtatttcctt acgaggcgaa gaaggacagg 3420

gtggcggtgc agcccagcag cctggagatg tcagcgctct ga 3462

<210> 2

<211> 1153

<212> PRT

<213> Homo sapiens

<400> 2

Met Ala Cys Pro Trp Lys Phe Leu Phe Lys Thr Lys Phe His Gln Tyr
1               5                   10                  15

Ala Met Asn Gly Glu Lys Asp Ile Asn Asn Asn Val Glu Lys Ala Pro
            20                  25                  30

Cys Ala Thr Ser Ser Pro Val Thr Gln Asp Asp Leu Gln Tyr His Asn
        35                  40                  45

Leu Ser Lys Gln Gln Asn Glu Ser Pro Gln Pro Leu Val Glu Thr Gly
    50                  55                  60

Lys Lys Ser Pro Glu Ser Leu Val Lys Leu Asp Ala Thr Pro Leu Ser
65                  70                  75                  80

Ser Pro Arg His Val Arg Ile Lys Asn Trp Gly Ser Gly Met Thr Phe
                85                  90                  95
Gln Asp Thr Leu His His Lys Ala Lys Gly Ile Leu Thr Cys Arg Ser
            100                 105                 110
Lys Ser Cys Leu Gly Ser Ile Met Thr Pro Lys Ser Leu Thr Arg Gly
        115                 120                 125
Pro Arg Asp Lys Pro Thr Pro Pro Asp Glu Leu Leu Pro Gln Ala Ile
    130                 135                 140
Glu Phe Val Asn Gln Tyr Tyr Gly Ser Phe Lys Glu Ala Lys Ile Glu
145                 150                 155                 160
Glu His Leu Ala Arg Val Glu Ala Val Thr Lys Glu Ile Glu Thr Thr
                165                 170                 175
Gly Thr Tyr Gln Leu Thr Gly Asp Glu Leu Ile Phe Ala Thr Lys Gln
            180                 185                 190
Ala Trp Arg Asn Ala Pro Arg Cys Ile Gly Arg Ile Gln Trp Ser Asn
        195                 200                 205
Leu Gln Val Phe Asp Ala Arg Ser Cys Ser Thr Ala Arg Glu Met Phe

    210                 215                 220

Glu His Ile Cys Arg His Val Arg Tyr Ser Thr Asn Asn Gly Asn Ile
225                 230                 235                 240

Arg Ser Ala Ile Thr Val Phe Pro Gln Arg Ser Asp Gly Lys His Asp
                245                 250                 255

Phe Arg Val Trp Asn Ala Gln Leu Ile Arg Tyr Ala Gly Tyr Gln Met
            260                 265                 270

Pro Asp Gly Ser Ile Arg Gly Asp Pro Ala Asn Val Glu Phe Thr Gln
        275                 280                 285

Leu Cys Ile Asp Leu Gly Trp Lys Pro Lys Tyr Gly Arg Phe Asp Val
    290                 295                 300

Val Pro Leu Val Leu Gln Ala Asn Gly Arg Asp Pro Glu Leu Phe Glu
305                 310                 315                 320

Ile Pro Pro Asp Leu Val Leu Glu Val Ala Met Glu His Pro Lys Tyr
                325                 330                 335

Glu Trp Phe Arg Glu Leu Glu Leu Lys Trp Tyr Ala Leu Pro Ala Val
            340                 345                 350

Ala Asn Met Leu Leu Glu Val Gly Gly Leu Glu Phe Pro Gly Cys Pro
        355                 360                 365
Phe Asn Gly Trp Tyr Met Gly Thr Glu Ile Gly Val Arg Asp Phe Cys
    370                 375                 380
Asp Val Gln Arg Tyr Asn Ile Leu Glu Glu Val Gly Arg Arg Met Gly
385                 390                 395                 400
Leu Glu Thr His Lys Leu Ala Ser Leu Trp Lys Asp Gln Ala Val Val
                405                 410                 415
Glu Ile Asn Ile Ala Val Leu His Ser Phe Gln Lys Gln Asn Val Thr
            420                 425                 430
Ile Met Asp His His Ser Ala Ala Glu Ser Phe Met Lys Tyr Met Gln
        435                 440                 445
Asn Glu Tyr Arg Ser Arg Gly Gly Cys Pro Ala Asp Trp Ile Trp Leu
    450                 455                 460
Val Pro Pro Met Ser Gly Ser Ile Thr Pro Val Phe His Gln Glu Met
465                 470                 475                 480
Leu Asn Tyr Val Leu Ser Pro Phe Tyr Tyr Tyr Gln Val Glu Ala Trp
                485                 490                 495

Lys Thr His Val Trp Gln Asp Glu Lys Arg Arg Pro Lys Arg Arg Glu
            500                 505                 510
Ile Pro Leu Lys Val Leu Val Lys Ala Val Leu Phe Ala Cys Met Leu
        515                 520                 525
Met Arg Lys Thr Met Ala Ser Arg Val Arg Val Thr Ile Leu Phe Ala
    530                 535                 540
Thr Glu Thr Gly Lys Ser Glu Ala Leu Ala Trp Asp Leu Gly Ala Leu
545                 550                 555                 560
Phe Ser Cys Ala Phe Asn Pro Lys Val Val Cys Met Asp Lys Tyr Arg
                565                 570                 575
Leu Ser Cys Leu Glu Glu Glu Arg Leu Leu Leu Val Val Thr Ser Thr
            580                 585                 590
Phe Gly Asn Gly Asp Cys Pro Gly Asn Gly Glu Lys Leu Lys Lys Ser
        595                 600                 605
Leu Phe Met Leu Lys Glu Leu Asn Asn Lys Phe Arg Tyr Ala Val Phe
    610                 615                 620
Gly Leu Gly Ser Ser Met Tyr Pro Arg Phe Cys Ala Phe Ala His Asp

625                 630                 635                 640

Ile Asp Gln Lys Leu Ser His Leu Gly Ala Ser Gln Leu Thr Pro Met

                645                 650                 655

Gly Glu Gly Asp Glu Leu Ser Gly Gln Glu Asp Ala Phe Arg Ser Trp

            660                 665                 670

Ala Val Gln Thr Phe Lys Ala Ala Cys Glu Thr Phe Asp Val Arg Gly

        675                 680                 685

Lys Gln His Ile Gln Ile Pro Lys Leu Tyr Thr Ser Asn Val Thr Trp

    690                 695                 700

Asp Pro His His Tyr Arg Leu Val Gln Asp Ser Gln Pro Leu Asp Leu

705                 710                 715                 720

Ser Lys Ala Leu Ser Ser Met His Ala Lys Asn Val Phe Thr Met Arg

                725                 730                 735

Leu Lys Ser Arg Gln Asn Leu Gln Ser Pro Thr Ser Ser Arg Ala Thr

            740                 745                 750

Ile Leu Val Glu Leu Ser Cys Glu Asp Gly Gln Gly Leu Asn Tyr Leu

        755                 760                 765

Pro Gly Glu His Leu Gly Val Cys Pro Gly Asn Gln Pro Ala Leu Val
    770                 775                 780

Gln Gly Ile Leu Glu Arg Val Val Asp Gly Pro Thr Pro His Gln Thr
785                 790                 795                 800

Val Arg Leu Glu Ala Leu Asp Glu Ser Gly Ser Tyr Trp Val Ser Asp
                805                 810                 815

Lys Arg Leu Pro Pro Cys Ser Leu Ser Gln Ala Leu Thr Tyr Phe Leu
            820                 825                 830

Asp Ile Thr Thr Pro Pro Thr Gln Leu Leu Leu Gln Lys Leu Ala Gln
        835                 840                 845

Val Ala Thr Glu Glu Pro Glu Arg Gln Arg Leu Glu Ala Leu Cys Gln
    850                 855                 860

Pro Ser Glu Tyr Ser Lys Trp Lys Phe Thr Asn Ser Pro Thr Phe Leu
865                 870                 875                 880

Glu Val Leu Glu Glu Phe Pro Ser Leu Arg Val Ser Ala Gly Phe Leu
                885                 890                 895

Leu Ser Gln Leu Pro Ile Leu Lys Pro Arg Phe Tyr Ser Ile Ser Ser
            900                 905                 910

Ser Arg Asp His Thr Pro Thr Glu Ile His Leu Thr Val Ala Val Val
        915                 920                 925
Thr Tyr His Thr Arg Asp Gly Gln Gly Pro Leu His His Gly Val Cys
    930                 935                 940
Ser Thr Trp Leu Asn Ser Leu Lys Pro Gln Asp Pro Val Pro Cys Phe
945                 950                 955                 960
Val Arg Asn Ala Ser Gly Phe His Leu Pro Glu Asp Pro Ser His Pro
                965                 970                 975
Cys Ile Leu Ile Gly Pro Gly Thr Gly Ile Ala Pro Phe Arg Ser Phe
            980                 985                 990
Trp Gln Gln Arg Leu His Asp Ser Gln His Lys Gly Val Arg Gly Gly
        995                 1000                1005
Arg Met Thr Leu Val Phe Gly Cys Arg Arg Pro Asp Glu Asp His Ile
    1010                1015                1020
Tyr Gln Glu Glu Met Leu Glu Met Ala Gln Lys Gly Val Leu His Ala
1025                1030                1035                1040
Val His Thr Ala Tyr Ser Arg Leu Pro Gly Lys Pro Lys Val Tyr Val

                1045                1050                1055

Gln Asp Ile Leu Arg Gln Gln Leu Ala Ser Glu Val Leu Arg Val Leu

            1060                1065                1070

His Lys Glu Pro Gly His Leu Tyr Val Cys Gly Asp Val Arg Met Ala

        1075                1080                1085

Arg Asp Val Ala His Thr Leu Lys Gln Leu Val Ala Ala Lys Leu Lys

    1090                1095                1100

Leu Asn Glu Glu Gln Val Glu Asp Tyr Phe Phe Gln Leu Lys Ser Gln

1105                1110                1115                1120

Lys Arg Tyr His Glu Asp Ile Phe Gly Ala Val Phe Pro Tyr Glu Ala

                1125                1130                1135

Lys Lys Asp Arg Val Ala Val Gln Pro Ser Ser Leu Glu Met Ser Ala

            1140                1145                1150

Leu

<210> 3

<211> 1152

<212> DNA

<213> Homo sapiens

<400> 3

aaaagtttga ccagaggacc cagggacaag cctacccctc cagatgagct tctacctcaa 60

gctatcgaat ttgtcaacca atattacggc tccttcaaag aggcaaaaat agaggaacat 120

ctggccaggg tggaagcggt aacaaaggag atagaaacaa caggaaccta ccaactgacg 180

ggagatgagc tcatcttcgc caccaagcag gcctggcgca atgccccacg ctgcattggg 240

aggatccagt ggtccaacct gcaggtcttc gatgcccgca gctgttccac tgcccgggaa 300

atgtttgaac acatctgcag acacgtgcgt tactccacca acaatggcaa catcaggtcg 360

gccatcaccg tgttccccca gcggagtgat ggcaagcacg acttccgggt gtggaatgct 420

cagctcatcc gctatgctgg ctaccagatg ccagatggca gcatcagagg ggaccctgcc 480

aacgtggaat tcactcagct gtgcatcgac ctgggctgga agcccaagta cggccgcttc 540

gatgtggtcc ccctggtcct gcaggccaat ggccgtgacc ctgagctctt cgaaatccca 600

cctgaccttg tgcttgaggt ggccatggaa catcccaaat acgagtggtt tcgggaactg 660

gagctaaagt ggtacgccct gcctgcagtg gccaacatgc tgcttgaggt gggcggcctg 720

gagttcccag ggtgcccctt caatggctgg tacatgggca cagagatcgg agtccgggac 780

ttctgtgacg tccagcgcta caacatcctg gaggaagtgg gcaggagaat gggcctggaa 840

acgcacaagc tggcctcgct ctggaaagac caggctgtcg ttgagatcaa cattgctgtg 900

ctccatagtt tccagaagca gaatgtgacc atcatggacc accactcggc tgcagaatcc 960

ttcatgaagt acatgcagaa tgaataccgg tcccgtgggg gctgcccggc agactggatt 1020

tggctggtcc ctcccatgtc tgggagcatc acccccgtgt ttcaccagga gatgctgaac 1080

tacgtcctgt cccctttcta ctactatcag gtagaggcct ggaaaaccca tgtctggcag 1140

gacgagaagc gg 1152

<210> 4

<211> 384

<212> PRT

<213> Homo sapiens

<400> 4

Lys Ser Leu Thr Arg Gly Pro Arg Asp Lys Pro Thr Pro Pro Asp Glu
1               5                   10                  15

Leu Leu Pro Gln Ala Ile Glu Phe Val Asn Gln Tyr Tyr Gly Ser Phe
            20                  25                  30

Lys Glu Ala Lys Ile Glu Glu His Leu Ala Arg Val Glu Ala Val Thr
        35                  40                  45

Lys Glu Ile Glu Thr Thr Gly Thr Tyr Gln Leu Thr Gly Asp Glu Leu
    50                  55                  60

Ile Phe Ala Thr Lys Gln Ala Trp Arg Asn Ala Pro Arg Cys Ile Gly
65                  70                  75                  80

Arg Ile Gln Trp Ser Asn Leu Gln Val Phe Asp Ala Arg Ser Cys Ser
                85                  90                  95

Thr Ala Arg Glu Met Phe Glu His Ile Cys Arg His Val Arg Tyr Ser
            100                 105                 110

Thr Asn Asn Gly Asn Ile Arg Ser Ala Ile Thr Val Phe Pro Gln Arg
        115                 120                 125
Ser Asp Gly Lys His Asp Phe Arg Val Trp Asn Ala Gln Leu Ile Arg
    130                 135                 140
Tyr Ala Gly Tyr Gln Met Pro Asp Gly Ser Ile Arg Gly Asp Pro Ala
145                 150                 155                 160
Asn Val Glu Phe Thr Gln Leu Cys Ile Asp Leu Gly Trp Lys Pro Lys
                165                 170                 175
Tyr Gly Arg Phe Asp Val Val Pro Leu Val Leu Gln Ala Asn Gly Arg
            180                 185                 190
Asp Pro Glu Leu Phe Glu Ile Pro Pro Asp Leu Val Leu Glu Val Ala
        195                 200                 205
Met Glu His Pro Lys Tyr Glu Trp Phe Arg Glu Leu Glu Leu Lys Trp
    210                 215                 220
Tyr Ala Leu Pro Ala Val Ala Asn Met Leu Leu Glu Val Gly Gly Leu
225                 230                 235                 240
Glu Phe Pro Gly Cys Pro Phe Asn Gly Trp Tyr Met Gly Thr Glu Ile
                245                 250                 255

Gly Val Arg Asp Phe Cys Asp Val Gln Arg Tyr Asn Ile Leu Glu Glu
            260                 265                 270
Val Gly Arg Arg Met Gly Leu Glu Thr His Lys Leu Ala Ser Leu Trp
        275                 280                 285
Lys Asp Gln Ala Val Val Glu Ile Asn Ile Ala Val Leu His Ser Phe
    290                 295                 300
Gln Lys Gln Asn Val Thr Ile Met Asp His His Ser Ala Ala Glu Ser
305                 310                 315                 320
Phe Met Lys Tyr Met Gln Asn Glu Tyr Arg Ser Arg Gly Gly Cys Pro
                325                 330                 335
Ala Asp Trp Ile Trp Leu Val Pro Pro Met Ser Gly Ser Ile Thr Pro
            340                 345                 350
Val Phe His Gln Glu Met Leu Asn Tyr Val Leu Ser Pro Phe Tyr Tyr
        355                 360                 365
Tyr Gln Val Glu Ala Trp Lys Thr His Val Trp Gln Asp Glu Lys Arg
    370                 375                 380

<210> 5

<211> 27

<212> DNA

<213> Artificial Sequence

<220>

<223> Description of Artificial Sequence:Artificially

Synthesized Sequence

<400> 5

aaaggtaccg agatggcctg tccttgg 27

<210> 6

<211> 20

<212> DNA

<213> Artificial Sequence

<220>

<223> Description of Artificial Sequence:Artificially

Synthesized Sequence

<400> 6

aagctatcga atttgtcaac 20

<210> 7

<211> 20

<212> DNA
<213> Artificial Sequence

<220>
<223> Description of Artificial Sequence:Artificially
      Synthesized Sequence

<400> 7
cacctgacct tgtgcttgag                                                    20

<210> 8
<211> 20
<212> DNA
<213> Artificial Sequence

<220>
<223> Description of Artificial Sequence:Artificially
      Synthesized Sequence

<400> 8
ttttgcctct ttgaaggagc                                                    20

<210> 9
<211> 20
<212> DNA
<213> Artificial Sequence

<220>

<223> Description of Artificial Sequence:Artificially

Synthesized Sequence

<400> 9

ccactcgtat ttgggatgtt 20

<210> 10

<211> 24

<212> DNA

<213> Artificial Sequence

<220>

<223> Description of Artificial Sequence:Artificially

Synthesized Sequence

<400> 10

cttgaagctt cgcttctcgt cctg 24

<210> 11

<211> 21

<212> DNA

<213> Artificial Sequence

<220>

<223> Description of Artificial Sequence:Artificially
      Synthesized Sequence

<400> 11
ggtaccgaat ctctggtcaa g                                                21

<210> 12
<211> 20
<212> DNA
<213> Artificial Sequence

<220>
<223> Description of Artificial Sequence:Artificially
      Synthesized Sequence

<400> 12
ccactcgtat ttgggatgtt                                                  20

## Description

### Technical Field

This invention relates to methods for screening inhibitors of inducible nitrogen oxide synthase activation (hereinafter, abbreviated as iNOS).

### Background Art

Nitric oxide (hereafter abbreviated as "NO"), which is a gaseous radical, was first reported as being identical to the endothelial cell derived vascular smooth muscle relaxing factor (EDRF) (Nature 327, 524-526, 1987). Thereafter, NO was found to have diverse physiological activities such as those shown below:
suppression of blood circulation
neurotransmission
suppression of platelet aggregation
bactericidal activity
carcinostatic activity
cytopathy

As the diverse physiological activities of NO became clear, it was thought that it might be possible to regulate diseases by regulating NO itself or NO production. For example, the effect of NO on improving the ventilatory function of lungs has been applied to inhalation therapy. Also, since NO is involved in cell damage and inflammatory symptoms, the regulation of NO production may be linked to the regulation of various pathologies.

*In vivo* NO is speculated to be produced by Nitrogen Oxide Synthase (EC: 1.14.13.39, hereinafter referred to as NOS) from arginine and oxygen as substrates. To date, the existence of the following three types of isozymes has been elucidated for NOS: endothelial constitutive NOS (hereinafter referred to as ecNOS), neural NOS (hereinafter referred to as nNOS), and inducible NOS (iNOS).

It is said that iNOS has the highest NO synthesizing ability among the three isozymes. On the other hand, ecNOS is localized in vascular endothelial cells. Besides in neurons, nNOS is constitutively expressed in skeletal muscles, renal macula densa, pancreatic β-cells, pulmonary epithelial cells, endometrial cells, and gastrointestinal brush cells. In contrast, iNOS is called the inducible form because it is induced by various cells upon treatment with TNF, IFN-γ, lipopolysaccharide (hereinafter, referred to as LPS) (J. Exp. Med. 176, 261-264, 1992), or such. Expression of iNOS has been reported in inflammatory macrophages, vascular smooth muscle cells, vascular endothelial cells, myocardial cells, hepatocytes, colon mucosal epithelial cells, pancreatic β-cells, glial cells, neurons, articular synovicytes, uriniferous tubule cells, mesangium cells, pulmonary epithelial cells, ovarian granulosa cells, and such.

The following reports have been made regarding the relationship of iNOS to pathology. First, the involvement of NO in graft rejection has been elucidated. During heart transplant rejection, iNOS expression is induced in macrophages accompanying an increase of cytokines such as IFN-γ, TNF-α, and IL-β. NO synthesized by iNOS inhibits systolic function of the heart and promotes apoptosis of the cardiac muscle, causing a decline of cardiac function and rejection. In such cases, inhibiting the function of iNOS has been reported to elevate the graft survival rate of the heart (Coronary Artery Disease 10,310-314,1999). Involvement of iNOS has also been speculated in the induction of neuronal cell death at infarction sites. For example, elevation of iNOS activity is observed at infarction sites of middle cerebral artery-ligated rats. This suggests that iNOS induces neuronal cell death (J. Cereb. Blood Flow Metab. 15, 52-59, 1995). Therefore, it may be possible to regulate these clinical condition by inhibiting iNOS activity.

Based on such a line of thought, a method of screening for compounds that inhibit the expression of iNOS was proposed (WO98/12313). In other words, it was an attempt to screen for compounds having expression regulating activity through a reporter assay using cells that have been transformed with vectors in which a signal gene has been inserted downstream of the 5'-flanking region of human iNOS. Since compounds that may be obtained by this screening have activity to suppress the expression of iNOS, these can be expected to have therapeutic effects on diseases such as the following: cardiac and cerebro-vascular disorder, ischemic heart disease, Septic shock, pain, rheumatism, arthritis, Asthma, immunodeficiency, viral or non-viral infection, Autoimmune disease, dementia, and cancer.

However, the construction of vectors and culturing of transformants that accompany reporter assays require sophisticated skills and also a long culture time to obtain results. Therefore, it is difficult to hope for a high efficiency in screening methods based on reporter assays.

Since an enormous number of candidate compounds, such as a combinatorial library, must be targets for screening in recent years, quickness is desired in such screenings. High throughput screening (HTS) is prevalent as a method for performing screening of such a large number of candidate compounds. However, since one cannot shorten the cultivation time by utilizing HTS, a screening method that does not require cell cultivation will be useful.

In addition, even if one could select some kind of candidate compound from this screening method, it may be difficult to expect swift action from that compound. Regulation of iNOS is not based on posttranscriptional modification of a protein and such, but is said to be based entirely on regulation at the transcriptional level (Cell 78, 915-918, 1994; Proc. Natl. Acad. Sci. USA. 90, 3539-3543, 1993). Therefore, the approach of regulating iNOS activity by compounds that regulate its expression may be considered rational. However, as long as the approach is based on the functional mechanism of inhibiting the expression, it may not be possible to regulate the activity of iNOS that already exists as a protein at the time of administration of that compound. Therefore, it will be useful if a screening method that can select compounds showing inhibitory effects by directly acting on the activation of iNOS that has been translated into the protein can be achieved.

On the other hand, the use of a compound that interferes with the catalyzed reaction may also be effective to accomplish inhibition of NOS activity. However, one can easily presume that such compounds will have inhibitory effects not only on iNOS, but also on other isozymes such as ecNOS and nNOS. By using iNOS expressing cells, compounds that suppress the function of iNOS can also be screened using NO production as an index. However, one cannot deny the possibility that compounds that can be obtained by such a screening may act on isozymes other than iNOS. In any case, it is difficult to expect a high selectivity towards iNOS. Therefore, a screening method for compounds that can specifically inhibit iNOS is desired.

Several compounds that function in an inhibitory manner towards iNOS and their mechanisms of inhibition have been elucidated. For example, it is considered that CO, NO, CN, imidazole, and N-phenyl imidazole that bind to heme all act on heme, which is a cofactor of iNOS, in order to inhibit its activation (J. Biol. Chem. 268, 9425-9429, 1993; Biochem. Biophys. Res. Commun. 193, 963-970, 1993; Biochemistry 34, 5627-5634, 1995; J. Biol. Chem. 270, 22997-23006, 1995; J. Biol. Chem. 269, 20335-20339, 1994).

iNOS is a homodimer composed of 130kDa subunits. Each subunit comprises an oxygenase domain and a reductase domain. In addition, it exhibits NOS activity only after binding of heme, biopterin, NADPH, FAD, FMN, and calmodulin. When binding of these cofactors are observed in more detail, NADPH, FAD, FMN, and calmodulin bind to the reductase domain. On the other hand, heme binds to the oxygenase domain, and furthermore, dimerization of the subunits is completed only after binding of biopterin (Biochemistry 34, 801-807, 1995). If any one of these factors is lacking, enzyme activity of iNOS is not expressed. Structural characteristics mentioned above can be shown as in Table 1.

**Table 1**

| | Oxygenase domain | Reductase domain |
|---|---|---|
| Region | N-terminal side | C-terminal side |
| | positions 1-508 | positions 509-1153 |
| Cofactor | Heme | NADPH |
| | Biopterin | FAD |
| | | FMN |
| | | Calmodulin |

Furthermore, it was elucidated that clotrimazole and miconazole, which were antifungal agents, interfered not only with enzyme activity of iNOS but also with its dimerization (J. Biol. Chem. 274/2, 930-938, 1999). Since these imidazole compounds have large molecular weights, steric hindrance that occurred due to their binding to heme was considered to interfere with iNOS homodimer formation, and hence the above phenomenon.

Therefore, compounds containing an imidazole ring are considered to be effective as inhibitors of iNOS. However, imidazole compounds are compounds that persistently bind to heme. Binding of iNOS to imidazole is accomplished via heme. Therefore, inhibitors based on the binding to heme may function in an inhibitory manner towards many proteins that use heme as a cofactor in a similar manner to that of iNOS. That is, if imidazole compounds are used as iNOS inhibitors, this may cause side effects when heme proteins other than iNOS coexist. Therefore, in order to obtain inhibitors specific for iNOS, inhibitors are desired whose sites of action are structures that are more specific to iNOS.

### Disclosure of the Invention

The objective of this invention is to provide screening methods for compounds that inhibit iNOS. More specifically, the objective of this invention is to provide screening methods for compounds that may inhibit dimerization of iNOS subunits.

First, the inventors elucidated the mechanism that inhibits iNOS. They considered that if the inhibition mechanism were elucidated, they would be able to design a screening method that could lead to the selection of a desirable compound. As a result, certain types of compounds having iNOS activity-inhibiting effects were found to specifically inhibit the dimerization of iNOS based on a mechanism different from the known dimerization inhibition mechanism. As mentioned earlier, some imidazole compounds are known to interfere with iNOS dimerization via binding to heme. However, it was found that when the compounds for which a novel inhibitory mechanism was found bind to iNOS, they do not substantially bind to heme, which is the binding site of imidazole compounds.

Based on these findings, the inventors found that compounds that inhibit iNOS dimerization can be screened by selecting compounds having a binding activity towards the iNOS monomer, or a mutant thereof, and thereby completed this invention. Furthermore, the inventors found that iNOS inhibitors can be provided by compounds that can be obtained by such screening methods, and thereby completed this invention. That is, this invention relates to the following screening methods and an iNOS inhibitor comprising as a main ingredient a compound that can be obtained by a screening method as the main ingredient.
[1] A method of *in vitro* screening for a compound that inhibits dimer formation of inducible nitrogen oxide synthase, wherein the method comprises the steps of:
   a) contacting candidate compounds with an inducible nitrogen oxide synthase monomer, or a mutant thereof,
   b) selecting a compound that binds to the inducible nitrogen oxide synthase monomer, or the mutant thereof.
[2] The method of [1], wherein the mutant of the inducible nitrogen oxide synthase monomer is a protein containing an amino acid sequence in which at least one amino acid selected from cysteine at position 110, lysine at position 113, and cysteine at position 115 has been deleted or mutated.
[3] The method of [2], wherein the cysteine at position 110 of the protein has been deleted or mutated.
[4] The method of [2], wherein the mutant of the inducible nitrogen oxide synthase monomer has amino acid residues up to position 122 on the N-terminal side in its oxygenase domain deleted or mutated.
[5] The method of any one of [2] to [4], wherein the mutant of the inducible nitrogen oxide synthase monomer is a protein that lacks the reductase domain.
[6] The method of [1], wherein the binding of the a candidate compound to the oxygenase domain monomer of inducible nitrogen oxide synthase, or to a mutant thereof, is detected by competitive inhibition of a binding ligand towards the oxygenase domain monomer, or the mutant thereof, by the candidate compound.
[7] The method of [6], wherein the ligand for competitive inhibition is a compound that inhibits dimerization of monomers by binding to the oxygenase domain of inducible nitrogen oxide synthase.
[8] The method of [7], wherein the ligand is a compound that does not bind to heme.
[9] An inhibitor of inducible nitrogen oxide synthase activation, wherein the inhibitor comprises as a main ingredient a compound that can be obtained by any one of the methods of [1] to [8].
[10] An agent for treating a disease selected from the group consisting of graft rejection following an organ transplant, cerebral infarction, and cardiovascular ischemia, wherein the agent comprises the inhibitor of [9].
[11] A protein according to any one of (a) to (c) below:
   (a) a protein comprising the amino acid sequence of SEQ ID NO: 4,
   (b) a monomeric stable protein having the activity to bind to a compound that inhibits dimerization by binding to inducible nitrogen oxide synthase, wherein the protein comprises an amino acid sequence wherein one or several amino acids in the amino acid sequence of SEQ ID NO: 4 has been replaced, deleted, inserted, and/or added, and
   (c) a monomeric stable protein having the activity to bind to a compound that inhibits dimerization by binding to inducible nitrogen oxide synthase, which is encoded by DNA that hybridizes under stringent conditions to DNA comprising the nucleotide sequence of SEQ ID NO: 3.
[12] A DNA encoding any one of the proteins of [11].
[13] An expression vector containing the DNA of [12].
[14] A transformant that harbors the DNA of [12] or the expression vector of [13] in an expressive manner.
[15] A method for producing the protein of [11], comprising cultivating the transformant of [14], and collecting an expression product from the transformant and/or its culture supernatant.
[16] A method for detecting the activity of a candidate compound to inhibit dimer formation of inducible nitrogen oxide synthase comprising the steps of:
   a) contacting the candidate compound with the protein of [11], and
   b) detecting the binding of the candidate compound to the protein.
[17] A method of screening for a compound that inhibits dimer formation of inducible nitrogen oxide synthase, comprising the step of selecting the candidate compound in which binding to the protein was detected in the method of [16].

Additionally, this invention relates to a method for inhibiting inducible nitrogen oxide synthase activity comprising the step of administering a compound obtainable by the method of [1] or [17]. Alternatively, this invention relates to a therapeutic method for any of the diseases selected from the group consisting of graft rejection following organ transplants, cerebral infarction, and cardiovascular ischemia, the method comprising the step of administering a compound obtainable by the method of [1] or [17].

Furthermore, this invention relates to the use of a compound obtainable by the method of [1] or [17] for producing an inhibitor of inducible nitrogen oxide synthase activity. Alternatively, this invention relates to the use of a compound obtainable by the method of [1] or [17] for producing a therapeutic agent for any one of the diseases selected from the group consisting of graft rejection following organ transplants, cerebral infarction, and cardiovascular ischemia.

This invention relates to methods of screening for compounds that inhibit dimerization of iNOS. Dimerization of iNOS is an important step in acquisition of enzyme activity. Therefore, compounds that inhibit iNOS dimerization are useful as inhibitors of iNOS. Compounds that inhibit enzyme activity through inhibition of dimerization act on the step following translation of iNOS to the protein. Therefore, rapid action can be expected compared to inhibitors that act on gene expression.

A screening method of this invention comprises the following steps.
a) contacting candidate compounds with the iNOS monomer, or a mutant thereof, and
b) selecting a compound that binds to the iNOS monomer, or to the mutant thereof.

In the screening methods of this invention, the candidate compounds are selected using the binding to the iNOS monomer, or the mutant thereof, as an index. In this invention, "iNOS analog" will be used as a term that includes the iNOS monomer, and mutants thereof. There are no limitations on the source of the iNOS analog used for the screening methods of this invention. In species other than humans, such as mice, iNOSs have been confirmed to be activated by dimerization (Biochemistry 34, 801-807, 1995). Normally, an iNOS derived from an animal species that is the target of administration of a compound obtained by screening is used. However, iNOSs that are mutually structurally similar may be used for screenings for different species. For example, mouse iNOS is 88% homologous to human iNOS, and can be used in screening for compounds that are to be administrated to humans. The structure of human iNOS and genes encoding it are already well known (Proc. Natl. Acad. Sci. USA, 90, 11419-23, 1993). iNOS necessary for the screening can be obtained by extraction from tissues and cultured cells. Otherwise, a gene encoding it can be expressed in an appropriate expression system, and obtained as a recombinant. The method to obtain a recombinant of iNOS is well known (Proc. Natl. Acad. Sci. USA, 90, 11419-23, 1993).

iNOS used for the screening of this invention should be used as a monomer. Since the objective is to screen for compounds having inhibitory activity towards dimerization through a binding assays, a molecule that has already formed a dimer would not be appropriate.

For the screening methods of this invention, not only the iNOS monomer, but also mutants of the iNOS monomer that fulfill the following two conditions may be used.
(1) The mutant itself can stably exist as a monomer.
(2) A compound that inhibits dimerization of natural iNOS monomers can bind to it.

In this invention, to "stably exist as a monomer" means that most of the iNOS mutant molecules can exist as monomers under experimental conditions. Therefore, as long as the binding ability to a compound that inhibits dimerization of natural iNOS monomers is not substantially lost, even if a portion of the molecules forms a dimer, they can be used as the iNOS mutant of this invention. Specifically, for example, when at least 50% or more, desirably 66% or more of all the molecules exist as a monomers, that protein can be said to stably exist as a monomer. On the other hand, examples of compounds inhibiting dimerization of natural iNOS monomers in this invention are compound A (a compound disclosed in WO96/16981) and such, which will be described later.

As especially preferred mutants of this invention, iNOS mutants comprising amino acid sequences in which amino acids necessary for dimerization have been deleted or mutated, can be indicated. For example, in the human iNOS oxygenase domain, at least one amino acid selected from among cysteine (C) at position 110, lysine (K) at position 113, and cysteine at position 115 play an important role in dimerization of monomers in natural iNOS. Human iNOS can be converted to a mutant that stably exists as a monomer by deleting or mutating any one of these three amino acids. Since C at position 110 in particular can be considered to play an important role in the dimerization, a monomer mutant in which at least C at position 110, and preferably also the remaining C and K are deleted or mutated is desired. For example, the iNOS mutant produced by the inventors, in which amino acids from the N-terminal end of the oxygenase domain to amino acid 122 have been deleted, stably exists as a monomer and binds to a compound that inhibits dimerization of natural iNOS. Therefore, for example, iNOS mutants in which amino acids from the N-terminal end up to position 122±10, and preferably up to position 122±5 have been deleted, are preferable as mutants used for the screening methods according to this invention.

These types of mutants can be obtained by introducing a mutation by PCR into a gene encoding iNOS, or by expressing it as a fragment lacking a portion of the gene. Otherwise, fragmentation can also be performed by digesting iNOS comprising a normal amino acid sequence with an amino acid sequence specific peptidase.

The iNOS monomer of this invention can also be obtained by inhibiting dimerization of a protein having the same structure as natural iNOS. As mentioned earlier, certain types of imidazole derivatives have been known to have the function of inhibiting iNOS dimerization. Examples of such imidazole derivatives are clotrimazol and miconazole. Therefore, by inhibiting iNOS dimerization with these imidazole derivatives, iNOS can be obtained as a monomer.

The purpose of the screening of this invention is to select compounds that inhibit dimerization. With a monomer in which dimer formation has been already inhibited by an imidazole derivative, one may worry that selection of the desired compound may not be carried out. However, findings by the inventors have confirmed that even compounds that do not bind to heme, which is the binding site of imidazole derivatives, bind to an iNOS monomer and interfere with its dimerization. Since such compounds have a binding activity towards monomers in which dimerization has been inhibited by imidazole derivatives, a screening based on this invention is possible. Therefore, a screening method of this invention can also be carried out using an iNOS monomer obtainable by inhibiting dimerization with an imidazole derivative.

As mentioned earlier, higher specificity for iNOS can be expected in compounds that can directly bind to iNOS, than in compounds that bind to iNOS via heme. Therefore, compounds that can bind to the iNOS monomer at a binding site different from that of imidazole derivatives are desirable as compounds that should be screened in this invention.

The iNOS monomer of this invention, or a mutant thereof, may be a protein that lacks the reductase domain. As mentioned earlier, iNOS comprises a reductase domain and an oxygenase domain. Among them, the reductase domain is not involved in iNOS dimerization. Therefore, even if that reductase domain is deleted, the deleted protein can be used for the screening of this invention.

In this invention, the binding of the candidate compound to the iNOS analog can be confirmed based on a known binding assay. Below, the principle of the binding assay that can be applied to this invention will be described.

### Principle 1: Competitive Inhibition

Binding activity of candidate compounds can be confirmed by using as an index, the competitive inhibition by a candidate compound of the binding of an iNOS analog to a compound that is known to bind to an iNOS monomer and then inhibit its dimerization. In this invention, a compound that clearly binds to an iNOS monomer and then inhibits its dimerization is sometimes called a ligand. Generally, any one of the three components, i.e., iNOS analog, ligand, and candidate compound, is labeled, and by immobilizing one of the remaining two components, a binding assay can be performed with ease. Labeling and immobilization can also be carried out indirectly using an avidin-biotin system, etc. Radioisotopes, fluorescent compounds, luminescent compounds, enzyme active substances, and such may be used for labeling. Methods to label proteins and low-molecular weight organic compounds with these labels are well known. An example of such an assay system is a system in which a labeled ligand and a candidate compound are reacted competitively with an immobilized iNOS analog.

To observe competitive inhibition, the ligand and the candidate compound are mixed, and both are contacted with the iNOS analog simultaneously. If the candidate compound binds to the iNOS analog, depending on its binding activity, the amount of ligand that binds to the iNOS analog decreases. Therefore, using the result obtained by performing a similar manipulation in the absence of a candidate compound as a control, the binding activity of the candidate compound towards the iNOS analog can be evaluated by comparing the amount of ligand that bound (or did not bind) to the iNOS analog. For example, in the example mentioned earlier, the amount of labeled ligand that binds (or does not bind) to the immobilized iNOS analog is affected by the binding activity of the candidate compound.

Compounds that can bind to iNOS and inhibit the dimerization of iNOS are used as ligands that may be used in this invention. Compounds that are especially preferable as ligands of this invention are those that directly bind to the iNOS analog without the mediation of heme. Such compounds can be selected from compounds having known iNOS inhibitor activity, such as that disclosed in WO96/16981. More specifically, compounds that are useful as ligands in this invention can be selected by the following steps;
i) selecting a compound that can bind to an iNOS analog by contacting the compound with the iNOS analog,
ii) confirming the inhibitory effect of the selected compound on iNOS dimerization.

Preferably, the following step is further conducted: iii) confirming that the binding of the selected compound to the iNOS monomer is substantively not inhibited by imidazole derivatives. This way, it is confirmed that the selected compounds bind to iNOS without mediating heme.

Binding of an iNOS analog to a compound to be selected can be easily confirmed by labeling one, immobilizing the other, and contacting the two with each other. On the other hand, inhibition of dimerization can be confirmed, for example, in the following manner. Namely, cultured cells in which iNOS production has been confirmed is cultured in the presence or absence of the compound to be selected. The presence of iNOS dimers within the two cell extracts are compared, and if iNOS dimer formation is inhibited when cultured in the presence of the compound, it can be confirmed that the compound inhibited dimerization. The iNOS dimer can be confirmed by gel filtration and such.

As cells that produce iNOS, RAW264.7 cells derived from mouse macrophage and such may be used. These cells can induce iNOS *in vitro* following stimulation with LPS and IFN-γ (Cancer Res. 47, 5590-4, 1987; Proc. Natl. Acad. Sci. 88, 7773-7, 1991). For example, the following compound A can be indicated as a ligand that may be used for the screening methods of this invention.

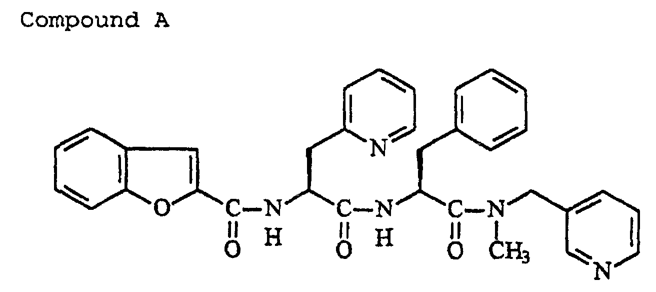

The iNOS analog can be labeled by known protein labeling methods. Otherwise, compound A can be obtained in its labeled form for example by reacting benzofuran-2-carboxylic acid with a radio-labeled compound shown below in formula (1). Benzofuran-2-carboxylic acid labeled with ¹⁴C -formula (2)- and ³H -formula (3)- can be obtained by known methods.

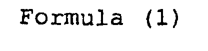

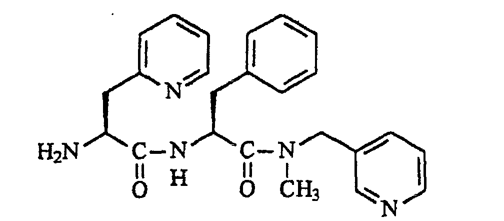

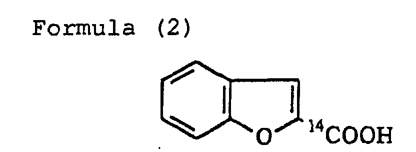

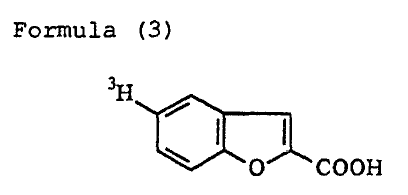

Patent applications have been submitted for all of the compounds disclosed in WO96/16981 as compounds having iNOS inhibitory activity. The inventors elucidated for the first time that the inhibitory activity is a result of the inhibition of dimer formation, and that the binding site is not heme, which was thought to be the binding site of imidazole derivatives. Therefore, using that binding activity as an index to screen compounds that can directly act on iNOS and inhibit iNOS activity more specifically is a novel finding made by the inventors.

On the other hand, an arbitrary compound can be used as a candidate compound that can be applied to a screening method of this invention. For example, natural compounds such as a protein library derived from a microorganism, plant, or animal cell may be used as candidate compounds. Also, it may be a library of artificial compounds obtained by combinatorial synthesis. Combinatorial libraries based on sugar chains, proteins, nucleic acids, or such are well known. These libraries can be synthesized chemically or biologically. Alternatively, a protein library presented by phage display can become candidate compounds.

### Principle 2: Binding Inhibition

Binding activity of a candidate compound can be confirmed using as an index, the candidate compound's interference with the binding of a ligand to the iNOS analog. Under this principle, first the candidate compound and the iNOS analog are contacted with each other, and then, a ligand is contacted. If the candidate compound can bind to the iNOS analog, the binding of the ligand (contacted later) to the iNOS analog is blocked.

### Principle 3: Replacement

Binding activity of a candidate compound can be confirmed based on the phenomenon of replacement of a ligand bound to the iNOS analog by a candidate compound. The order of contact of the two will be the reverse of that in binding inhibition.

### Principle 4: Direct Binding

Binding activity of a candidate compound can be confirmed by contacting the candidate compound with the iNOS analog, then using their binding as an index. When using the principle of direct binding, screening can be performed easily by labeling one and immobilizing the other.

On the other hand, the absence of a binding of a candidate compound to heme in this invention can be confirmed by a principle similar to that of the binding assay with the iNOS analog. For a binding assay to monitor the binding with heme, heme and a compound known to bind to it can be used. Examples of such compounds may be imidazole compounds such as clotrimazole and miconazole. That is, if the binding of the two is not substantially inhibited by a candidate compound, one can say that the candidate compound does not substantially bind to heme. More specifically, that candidate compound is judged not to have a substantial binding activity towards heme when the activity to bind to heme is for example 1/100 or less, and desirably 1/1000 or less than that of imidazoles.

In addition, this invention provides a mutant of the iNOS monomer useful for an aforementioned screening method. That is, this invention relates to any one of the following proteins of (a) to (c);
(a) a protein comprising the amino acid sequence of SEQ ID NO: 4,
(b) a protein comprising an amino acid sequence in which one or several amino acids in the amino acid sequence of SEQ ID NO: 4 have been replaced, deleted, inserted, and/or added, has the activity to bind to compounds that inhibit dimerization by binding to iNOS, and is also stable as a monomer, and
(c) a protein encoded by DNA that hybridizes under stringent conditions to DNA comprising the nucleotide sequence of SEQ ID NO: 3, has an activity to bind to a compound that inhibits dimerization by binding to iNOS, and is also stable as a monomer.

The iNOS mutant according to this invention, for example (a) a protein comprising the amino acid sequence of SEQ ID NO: 4, is encoded by a fragment in which the region corresponding to position 1-122 from the N-terminal end (positions 1-366 from the 5'-end of ORF) of the oxygenase domain is removed from the open reading frame (positions 1-3462 of ORF) of iNOS indicated in SEQ ID NO: 1 (GenBank Acc. No. X73029), and furthermore the reductase domain (positions 1579-3462 from the 5'-end of ORF) is removed. Structure of the gene encoding iNOS is already known (Proc. Natl. Acad. Sci. USA, 90, 11419-23, 1993).

In addition, (b) a protein that contains mutations in the amino acid sequence of the protein of (a) and is functionally equivalent to (a) are included in the iNOS mutant of this invention. In this invention, "functionally equivalent" means that the mutant is a protein having the activity to bind to a compound that inhibits dimerization by binding to iNOS, and is also stable as a monomer. A protein that is functionally equivalent to the protein of (a) can be obtained by introducing a mutation into the nucleotide sequence of the aforementioned ORF. A method to introduce mutations randomly into a given nucleotide sequence, or specifically into a specified region is well known. In addition, based on the aforementioned method, one can confirm that the obtained mutant has a binding activity towards a compound that inhibits dimerization by binding to iNOS, and is a protein that is also stable as a monomer.

Furthermore, the iNOS mutant of this invention may also be (c) encoded by DNA that hybridizes under stringent conditions to DNA comprising the nucleotide sequence of SEQ ID NO: 3, and include a protein that is functionally equivalent to the protein of (a). The nucleotide sequence of SEQ ID NO: 3 encodes the protein of (a). There is a strong possibility that DNA hybridizing under stringent conditions to DNA comprising this nucleotide sequence encodes a protein that is structurally similar to that of (a). Therefore, by selecting a protein that is structurally similar to (a) and is functionally equivalent to (a), the selected protein may be used for the screening methods of this invention. In this invention, stringent conditions can indicate, for example, the following conditions. Stringency varies depending on conditions such as temperature, denaturant, or salt concentration. Conditions that provide a similar stringency can be set by one skilled in the art according to experience.
Buffer: 5x SSC solution, 5x Denhardt solution, 50 mM sodium pyrophosphate (pH 6.5), 0.1% SDS, 50% formamide
Conditions for hybridization: 42°C, 2 to 16 hours
Washing conditions: 0.1 to 2x SSC, 0.1% SDS
Washing temperature: 22°C to 65°C

This invention relates to DNA encoding these iNOS mutants. The DNA of this invention includes DNA comprising the nucleotide sequence of SEQ ID NO: 3, and in addition, DNA that hybridizes to this DNA and encodes a protein that is functionally equivalent to the protein of (a) . Furthermore, the DNA of this invention includes DNA comprising various nucleotide sequences that encode the amino acid sequence of SEQ ID NO: 4. In addition, this invention includes DNA encoding a protein that is functionally equivalent to that of (a) and comprising an amino acid sequence in which one or several amino acids in the amino acid sequence of SEQ ID NO: 4 have been replaced, deleted, inserted, and/or added

The iNOS mutants of this invention can be obtained as recombinants for example by the following method using the aforementioned DNA and known expression systems. There are no particular limitations on the host cells, but bacteria can be given as examples. Examples of bacteria are bacterial strains belonging to genus *Escherichia*, (for example, *E. coli* JM109 ATCC 53323, E. *coli* HB101 ATCC 33694, *E. coli* MN102, *E. coli* HB101-16 FERM BP-1872, *E. coli* 294 ATCC 31446, and such), bacterial strains belonging to genus *Bacillus (Bacillus subtilis*, etc.), and so on. For example, the bacterial strain belonging to genus *Escherichia*, specifically *E. coli* HB101, *E. coli* JM109, or such are preferably used. In addition, when expressing a protein using an expression vector system in which a T7 promoter has been inserted, *E. coli,* or such (for example, ER2566 and such from NEB), which has DNA encoding T7 RNA polymerase inserted into its genome, is preferred.

When bacteria, especially *E. coli* is used as a host cell, usually, the expression vector is composed of at least a promoter-operator region, initiation codon, DNA encoding the amino acid sequence of the iNOS mutant of this invention, stop codon, terminator region, and replicative unit.

The promoter-operator region comprises a promoter, operator, and Shine-Dalgarno (SD) sequence (for example, AAGG and such). Preferably, the promoter-operator region may include a commonly used promoter-operator region (for example, PL-promoter, trp-promoter, T7-promoter, or such of *E. coli*). An example of a preferable initiation codon is the methionine codon (ATG).

DNA encoding an iNOS mutant of this invention can be prepared by known methods. For example, using a DNA synthesizer, a portion or the entire DNA comprising the desired nucleotide sequence can be synthesized. Alternatively, it can also be synthesized by using an mRNA or cDNA library of cells expressing iNOS as a template, and using a primer that is set based on the nucleotide sequence shown in SEQ ID NO: 3. Using a primer comprised of a nucleotide sequence to which artificial mutations have been made, a protein having a mutation in its amino acid sequence can be obtained. If a nucleotide sequence encoding a histidine tag is added to a primer, a fusion protein with a His tag can also be expressed. Alternatively, by linking a gene encoding a protein such as GST to the iNOS gene, a fusion protein of the two can be obtained. Vectors useful for expression of these fusion proteins are well known. A fusion protein with a His tag or GST can be easily purified by utilizing the binding affinity to the corresponding ligand.

Commonly used stop codons (for example TAG, TGA, etc.) can be indicated as examples of the stop codon. Natural or synthetic terminators (for example synthetic fd phage terminator, etc.) can be given as examples of the terminator region.

Replicative units refer to DNA compounds having the ability to replicate the entire DNA sequence in a host cell, and includes natural plasmids, artificially modified plasmids (for example, a DNA fragment prepared from a natural plasmid), and synthetic plasmids. Examples of preferable plasmids among *E. coli* are pBR322 plasmid, its artificially modified products (DNA fragments that are obtained by treating pBR322 with an appropriate restriction enzyme) , or such.

The expression vector can be prepared by linking a promoter, initiation codon, DNA encoding the amino acid sequence of the iNOS mutant of this invention, stop codon, and terminator region to an appropriate replicative unit (plasmid) in a successive and cyclic manner. An example is pET vector (Novagen), etc. In this case, if desired, an appropriate DNA fragment (for example, linkers, other restriction sites, etc.) can also be used according to common methods (for example, digestion by a restriction enzyme, and ligation using T4 DNA ligase).

By introducing a vector constructed in this manner into a host cell, a transformant (transfectant) can be prepared. Introduction of the expression vector into the host cell [transformation (transfection)] can be performed using a conventionally known technique (for example, Kushner method in the case of *E. coli*). The iNOS mutant of this invention can be produced by cultivating a transformant containing an expression vector prepared as described above in a nutrient medium.

The nutrient medium may contain a carbon source (for example, glucose, glycerol, mannitol, fructose, and lactose), and inorganic nitrogen or organic nitrogen source (for example ammonium sulfate, ammonium chloride, hydrolysate of casein, yeast extract, polypeptone, bactopeptone, and beef extract). Also, if desired, other nutrient sources and selection agents such as the following may be added.
Inorganic salts
   sodium diphosphate or potassium diphosphate
   dipotassium hydrogenphosphate
   magnesium chloride
   magnesium sulfate
   calcium chloride
   vitamins (for example, vitamin B1), and such
Antibiotics
   ampicillin
   kanamycin
   aminolevulinic acid, and such

Transformants (transfectants) are cultured by a method known in the art. Culture conditions, for example, temperature, pH of the medium, and time are selected so that the recovered amount of the desired iNOS mutant will be largest. Normally, the culture is carried out at pH 5.5 to 8.5 (preferably pH 7 to 7.5), at 5 to 40°C (preferably 10 to 30°C), and for 5 to 50 hours.

Transformed host cells grow in the medium, and the iNOS mutant can be collected from the host cells and/or its culture supernatant. When *E. coli* is used as a host, the iNOS mutant of this invention exists in the periplasm or cytoplasm of the normally cultured transformant. Therefore, these peptides can be obtained, for example, by the following method.

First, cells are collected by standard methods such as filtration and centrifugation, and a cell lysate is obtained by treating the cell wall and/or cell membrane with an appropriate combination of ultrasonication, freeze thawing, lysozyme, or such. Next, the obtained cell lysate is dissolved in an appropriate aqueous solution. For example, the following lysis buffer can be used as the aqueous solution used in this case.

| Lysis Buffer | |
|---|---|
| PMSF 1 mM | EDTA 0.5 mM |
| DTT 1 mM | Pepstatin 5 µg/ml |
| Aprotinin 5 µg/ml | Leypeptin 5 µg/ml |

These are dissolved in 50 mM Tris-HCl buffer (pH 7.5).

Then from the solution, an iNOS mutant of this invention is isolated and purified according to standard methods generally used to purify and isolate natural or synthetic proteins. Examples of isolation and purification methods are dialysis, gel filtration, affinity column chromatography using monoclonal antibodies against an iNOS mutant of this invention, column chromatography through an appropriate adsorbent, high-performance liquid chromatography, and such.

By using a mutant of the iNOS monomer according to this invention, activity of the candidate compound to inhibit dimerization of the iNOS monomer can be detected. That is, this invention relates to a method for detecting the activity to inhibit dimerization of iNOS, in which the method includes the following steps;
a) contacting a candidate compound with the aforementioned mutant of the iNOS monomer
b) detecting the binding of the candidate compound to the aforementioned protein.

Furthermore, utilizing this detection method, a screening method of this invention can be carried out. That is, this invention relates to a screening method for compounds that inhibit dimer formation of iNOS, and comprises the step of selecting candidate compounds in which binding to the protein has been detected in the aforementioned detection method.

In addition, this invention relates to an activation inhibitor of the aforementioned enzyme that inhibits dimerization by binding to iNOS, containing compounds that can be obtained by the aforementioned screening method as a major ingredient. Since the activation inhibitor of iNOS of this invention acts specifically on iNOS, and inhibits the final step of its activation, high selectivity and rapid action can be expected towards iNOS. In particular, absence of binding to heme, which is the binding site on iNOS for imidazole derivatives, means that it can be an inhibitor that binds by recognizing the structure of iNOS itself. In such inhibitors, an especially high selectivity can be expected.

The activation inhibitor of iNOS according to this invention is useful for regulating many diseases and pathologies brought forth by activation of iNOS. Specifically, for example, an iNOS inhibitor according to this invention can be used for regulating diseases and pathologies such as those shown below. Among them, iNOS has been shown, for example, to be involved in graft rejection following organ transplants and cerebral infarction. Therefore, it is possible to regulate such pathologies by inhibiting iNOS.

| | |
|---|---|
| adult respiratory distress syndrome | |
| cardiovascular ischemia | |
| myocarditis | heart failure |
| synovitis | |
| shock symptoms such as septic shock | |
| diabetes such as insulin-dependent diabetes | |
| diabetic nephropathy | |
| diabetic retinopathy | diabetic neuropathy |
| nephritis | peptic ulcer |

inflammatory enteropathy such as ulcerative colitis, and chronic colitis

| | |
|---|---|
| cerebral infarction | cerebral ischemia |
| migrine | chronic rheumatoid arthritis |
| gout neuritis | neuritis |
| postherpetic neuralgia | osteoarthritis |
| osteoporosis | systemic lupus erythematosus |
| organ transplant rejection | asthma |
| metastasis | Alzheimer's disease |
| arthritis | central neuropathy |

An iNOS inhibitor according to this invention can be administered as a single compound or as a mixture, in a pure or impure form, preferably by incorporating into a filling agent or carrier used for pharmaceutical formulations. The iNOS inhibitor of this invention comprises as an active ingredient a compound that can be obtained by a screening of this invention, and can be formulated by mixing with organic or inorganic carriers, or with filling agents appropriate for external (local), enteral, intravenous, intramuscular, parenteral, or intramucosal applications. The iNOS inhibitor formulations based on this invention can be used, for example, in the solid, semi-solid, or liquid form. The active ingredient can be combined with pharmaceutically acceptable commonly used non-toxic carriers that are used in a form suited for use, for example, as ointments, creams, plasters, tablets, pellets, encapsulated formulations, suppositories, liquid formulations (for example liquid saline), emulsions, suspensions (for example olive oil), aerosols, pills, powders, syrups, infusions, troches, cataplasms, aromatic water, lotions, buccal formulations, sublingual administration agents, intranasal administration agents, and such. Carriers that can be used are water, wax, glucose, lactose, gum from Acacia, gelatin, mannitol, starch paste, magnesium trisilicate, talc, cornstarch, keratin, paraffin, colloidal silica, potato starch, urea, and other solid, semi-solid, or liquid carriers suited for use in producing formulations, furthermore, adjuvants, stabilizers, thickeners, coloring agents, and fragrances may be used. An effective amount of active compounds sufficient for having a desired effect on the progress or state of a disease are included in the pharmaceutical composition.

The pharmaceutical agent of this invention can be administered, for example, by oral administration, injection, external application, inhalation, and intramucosal administration.

Examples of mammals that can be treated according to this invention are livestock, bred mammals such as cattle and horses, domesticated animals such as dogs, cats, and rats, and humans, but humans are preferred.

The therapeutically effective dose of an iNOS inhibitor included in the pharmaceutical agent of this invention changes with and depends on the age and condition of each patient who is to be treated, but for systemic applications, administration of a daily dose of approximately 0.01 to 1000 mg, preferably 0.1 to 500 mg, and more preferably 0.5 to 100 mg of the active ingredient for disease treatment are common, and generally, on average, approximately 0.2 to 0.5 mg, 1 mg, 5 mg, 10 mg, 50 mg, 100 mg, 250 mg, and 500 mg are administered in one dose. For long-term administration to humans, the preferred daily dose is within the range of approximately 0.3 mg/person to 1000 mg/person.

All publications of prior art referred to in this description are incorporated by reference into this description.

### Brief Description of the Drawings

Fig. 1 is a set of graphs showing the radioactivity in each of the fractions obtained from a gel filtration chromatography of a cell extract of mouse macrophage derived RAW264.7 cells. Fig. 1a shows the radioactivity when LPS/IFN is added, and Fig. 1b shows the radioactivity when nothing is added.
Fig. 2 is a set of graphs showing the relationship between the amount of ¹⁴C-A added to the medium and the radioactivity.
Fig. 3 is a set of graphs showing dot blotting by iNOS antibodies and ¹⁴C-A binding activity of the gel filtration fractions. Fig. 3a shows the result of dot blotting with LPS/IFN stimulation alone, Fig. 3b shows the result of dot blotting when compound A is added to the conditions of Fig. 3a, and Fig. 3c is a graph indicating the radioactivity.
Fig. 4 is a graph showing the binding of 14C-compound A to 2',5'-ADP Sepharose 4B, and dissociation of activity due to trypsin.
Fig. 5 is a set of graphs showing the result of radioactivity measurements of each fraction after separation by gel filtration chromatography using 2',5'-ADP Sepharose. The graph on the top is before trypsin treatment, and the graph at the bottom is after trypsin treatment.
Fig. 6 is a photograph of a gel electrophoresis showing restricted hydrolysis of *E. coli*-expressed iNOS by trypsin.
   Lane 1: 0.5 hours of Try treatment, 2 hours after 4 M urea treatment
   Lane 2: 2 hours of 4 M urea treatment, 0.5 hours after Try treatment
   Lane 3: before trypsin digestion
   Lane M: molecular weight marker
Fig. 7 is a photograph of a gel electrophoresis indicating the purification process by Ni-NTA agarose of *E. coli*-expressed Δ122-iNOSox.
Fig. 8 is a diagram indicating the binding saturation curve of Δ122-iNOSox and 3H-compound A.
Fig. 9 is a diagram indicating the Scatchard plot (correlation coefficient of 0.989) of Δ122-iNOSox and 3H-compound A.
Fig. 10 is a diagram indicating the result of analysis by Hill plot (correlation coefficient 0.989).

### Best Mode for Carrying out the Invention

Next, this invention will be described in more detail based on examples.

### [Example 1] Cultivation of mouse macrophage derived RAW264.7 cells and preparation of a cell extract

Mouse macrophage derived RAW264.7 cells (Stuehr D. J. Proc. Natl. Acad. Sci. USA. 1991, 88, 7773; Cancer Res. 1987, 47, 5590) were cultured in Dulbecco's Modified Eagle Medium containing 10% FCS (fetal calf serum), until there were 2 to 4x 10⁷ cells in each F75 culture flask (Falcon). Then, lipopolysaccharide (LPS) and interferon-γ (IFN) were added to a final concentration of 4 µg/ml and 16 units/ml, respectively, and cultured overnight in a CO₂ incubator (37°C). 0.1% Trypsin (5 mL) was added to the flask in which the cells were cultured, the cells were detached by an incubation at 37°C for 5 minutes, and then collected by centrifugation. To the collected cells (2,000 rpm, 10 minutes), 600 µL of lysis buffer was added, and freeze thawing was repeated three times to disrupt the cells. The supernatant obtained by centrifugation (15,000 rpm, 15 minutes) was collected and this was the cell extract.

| Dulbecco's Modified Eagle Medium | |
|---|---|
| L-arginine-HCl 84.0 mg/L | Na₂ L-cysteine 56.8 mg/L |
| L-histidine-HCl.H₂O 42.0 mg/L | L-isoleucine 104.8 mg/L |
| L-leucine 104.8 mg/L | L-lysine-HCl 146.2 mg/L |
| L-methionine 30.0 mg/L | L-phenylalanine 66.0 mg/L |
| L-serine 42.0 mg/L | L-threonine 95.2 mg/L |
| L-tryptophan 16.0 mg/L | L-tyrosine 72.0 mg/L |
| L-valine 93.6 mg/L | glycine 30.0 mg/L |
| choline chloride 4.0 mg/L mg/L | calcium D-pantothenate 4.0 |
| folic acid 4.0 mg/L | i-inositol 7.0 mg/L |
| nicotinamide 4.0 mg/L | pyridoxal-HCl 4.0 mg/L |
| riboflavin 0.4 mg/L | thiamine-HCl 4.0 mg/L |
| Na pyruvate 110.0 mg/L | dextrose 4500 mg/L |

### [Example 2] Detection of ¹⁴C-A binding activity

Compound A labeled with ¹⁴C (0.018 Ci/mmol, hereinafter referred to as ¹⁴C-A) was added at a final concentration of 10⁻⁶ M, 30 minutes before addition of LPS and IFN in Example 1 to prepare a cell extract.

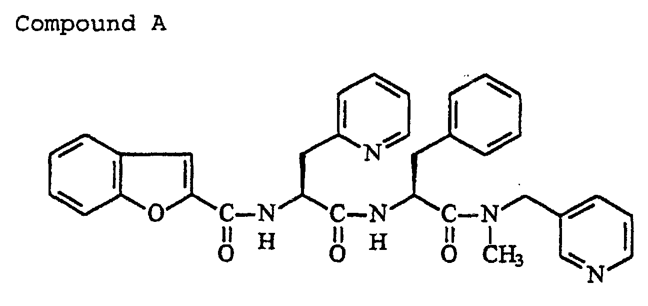

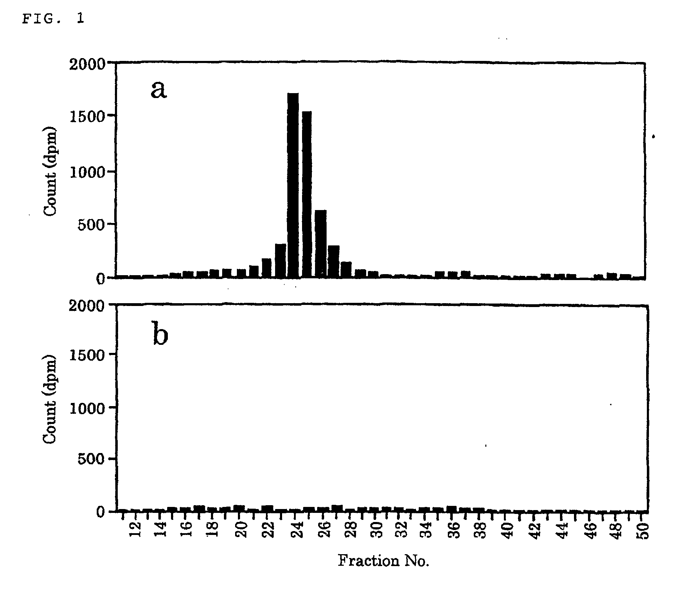

Then, 200 µL of that extract was applied to Superdex 200 HR10/30 (flow rate 0.5 mL/min: Amersham Pharmacia Biotech, Sweden) pre-equilibrated with 25 mM Tris-HCl (pH 8.0) containing 150 mM NaCl to perform gel filtration chromatography. Each of the fractions collected every minute were mixed with 7 mL of Aquasol-2 (Packard, USA), and was then measured with a liquid scintillation counter (Fig. 1). ¹⁴C-A used in this experiment was synthesized as follows.

That is, it was synthesized using a compound having the structure shown in Formula (1), and a ¹⁴C-labeled benzofuran-2-carboxylic acid shown in Formula (2) according to a known method (WO96/16981).

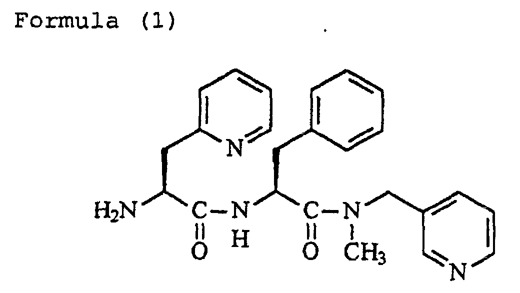

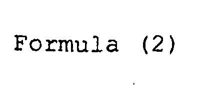

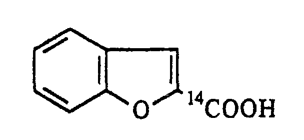

As a result, as shown in Fig. 1-a, elution of radioactivity was confirmed in a single peak in which the apex was at fraction (fr.) 23 and 24. In contrast, absolutely no radioactivity was confirmed from the cell extract prepared without addition of LPS/IFN (cells in which iNOS was not induced) (Fig. 1-b).

### [Example 3] Dose dependency of ¹⁴C-A binding activity

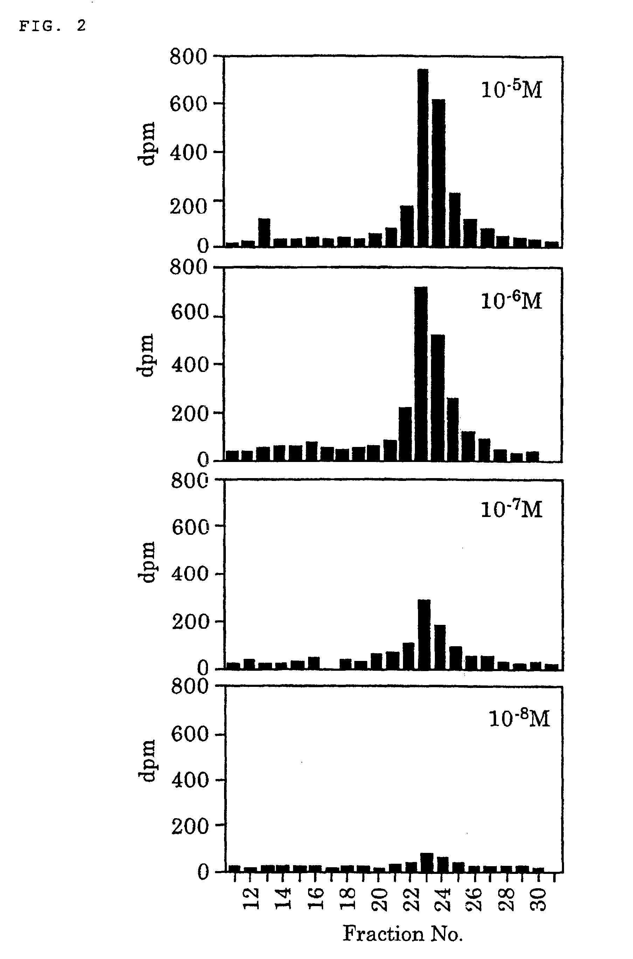

In the experiment of Example 2, when radioactivity of a cell extract prepared by changing the concentration of added ¹⁴C-A was observed, increase in radioactivity dependent on the added amount of ¹⁴C-A was confirmed between 10⁻⁸ M and 10⁻⁶ M as shown in Fig. 2, furthermore, saturation of radioactivity was confirmed between 10⁻⁶ M and 10⁻⁵ M. This radioactivity was considered to be derived from a ¹⁴C-A-bound protein in RAW cells.

### [Example 4] Blotting of gel filtration fractions containing ¹⁴C-A binding activity

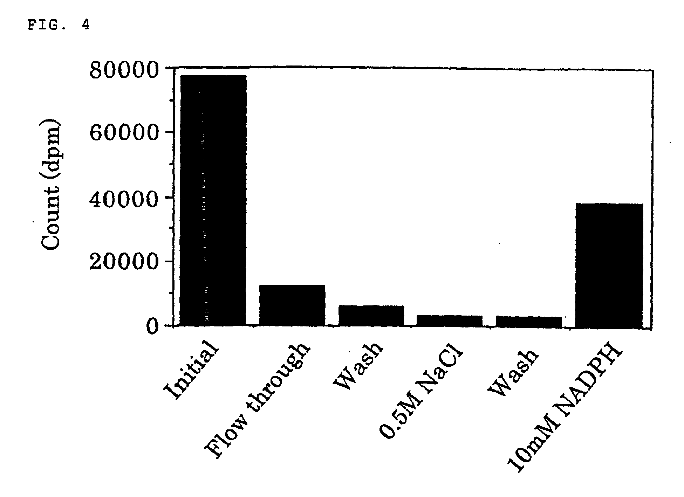

Gel filtration fractions containing compound A binding activity, which were obtained by a method similar to that of Example 2 were analyzed by dot blotting. That is, 200 µL each of the gel filtration fractions were taken and the protein component was immobilized using a dot blotting apparatus (Bio-Rad Laboratories, USA) equipped with a polyvinylidiene fluoride membrane (PVDF membrane; Millipore, USA). The PVDF membrane was stained with the ECL Western Blotting Detection System (Amersham Pharmacia Biotech, Sweden) using anti-iNOS antibody (1:1500 dilution; Santa Cruz Biotechnology, USA) as the primary antibody, and peroxidase labeled anti-rabbit IgG antibody (goat) as the secondary antibody, and detection of the iNOS protein was performed. The intensity of color of the stained dots was digitized by an analysis with computer imaging (NIH Image) , and was graphed. The results are shown in Fig. 4.

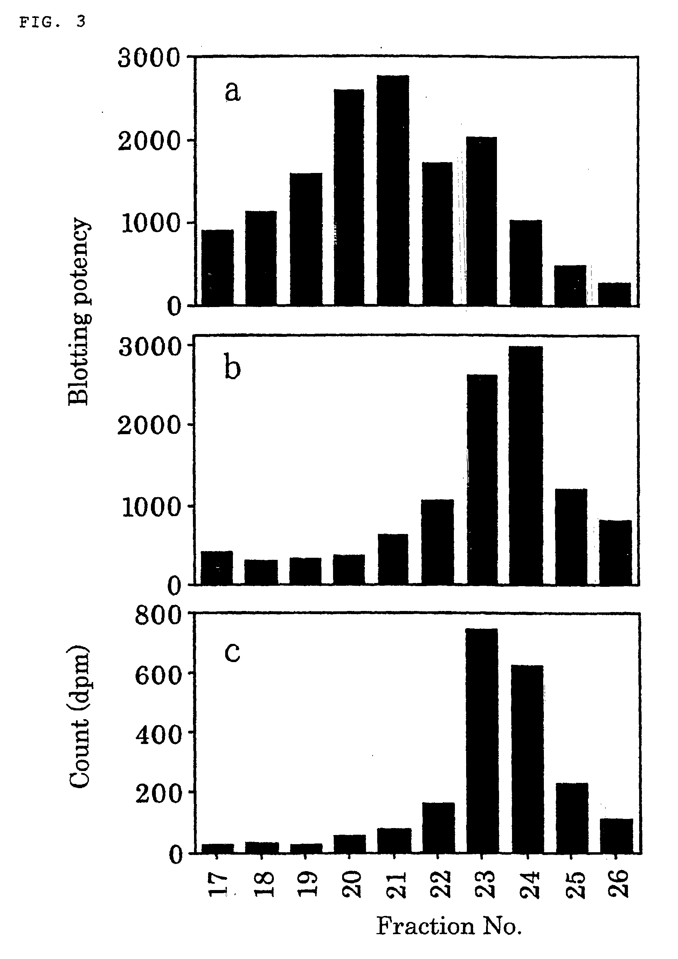

Dot blotting of a sample prepared by performing LPS/IFN stimulation alone without addition of compound A (Fig. 3a) yielded two peaks (Fr. 20, 21 and Fr. 23, 24), and they were considered to correspond to the dimer and monomer of iNOS, respectively. In contrast, in the case of a sample prepared by adding compound A, the peak corresponding to the dimer had clearly disappeared, and only the peak corresponding to the monomer was confirmed (Fig. 3b). Also, this peak was found to match the radioactivity obtained in Example 2 (Fig. 3c). That is, the iNOS monomer peak detected by dot blotting and ¹⁴C-A binding activity were judged to be the same in terms of gel filtration chromatography.

The results mentioned above suggest that compound A suppresses activation of iNOS by binding to the iNOS monomer and thereby almost completely suppresses its dimerization.

### [Example 5] Identification of ¹⁴C-A-binding protein

In Example 4, a result suggesting that ¹⁴C-A is an iNOS monomer molecule was obtained, and in order to prove this, the binding protein was purified using the radioactivity of ¹⁴C-A as an index. As a result of purification by 2',5'-ADP Sepharose 4B and DEAE-Sephacel (Amersham Pharmacia), binding radioactivity prepared according to Example 2 was confirmed as almost a single band of approximately 130 kDa by SDS-PAGE. This protein molecule was recognized by anti-iNOS antibody (Upstate Biotechnology) by Western blotting, and furthermore, it was confirmed that a partial amino acid sequence identified by In Gel Digestion method (Analytical Biochemistry, 224, 451-455 (1995)) matched the partial sequence of iNOS derived from mice (GenBank Acc. No. M84373).

### [Example 6] Binding of ¹⁴C-A binding active fraction to 2',5'-ADP Sepharose 4B and dissociation of its fraction by trypsin

iNOS is composed of two domain structures . One is an oxygenase domain, and the other is a reductase domain. D. K. Ghosh et al. (Ghosh D. K. and Stuehr D. J. 1995, Biochemistry 34: 801-807) succeeded in specific cleavage of a peptide bond between the two domains by treating the active form of iNOS (dimer) with trypsin, furthermore, they adsorbed the reductase domain alone using 2',5'-ADP Sepharose 4B, and reported that the two domains could be collected separately. Therefore, to elucidate which of the two domains of iNOS compound A binds to, the following experiment was performed utilizing the findings by Ghosh et al.

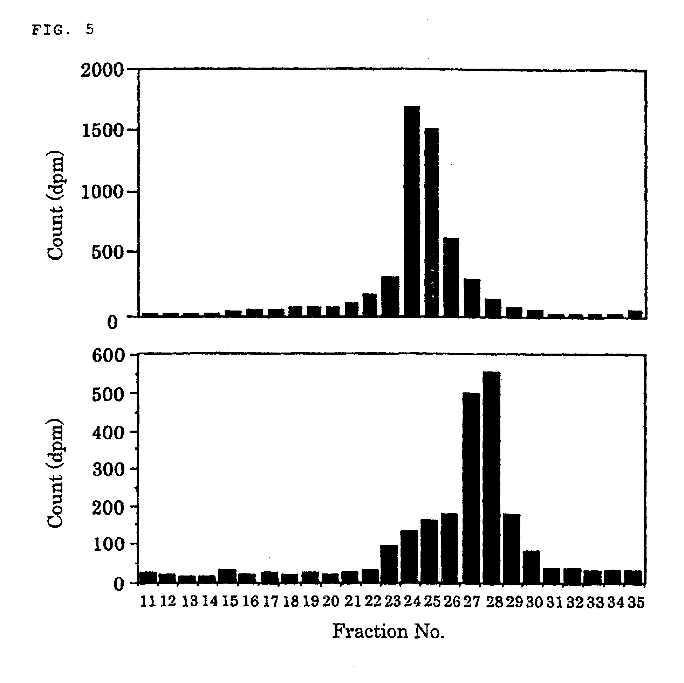

Binding radioactivity-comprising RAW cell extract prepared by the method shown in Example 2 was mixed with 2',5'-ADP Sepharose 4B gel (Amersham Pharmacia Biotech) equilibrated with 25 mM Tris-HCl (pH 7.5) containing 10% glycerol and 3 mM DTT, and was shaken for approximately 30 minutes at room temperature. The supernatant was collected by low-speed centrifugation (1000 rpm, 30 seconds), and when its radioactivity was measured, approximately 50% of radioactivity adsorbed on the gel was recovered (Fig. 4). Next, gel filtration chromatography was performed on the supernatant containing the radioactivity without further treatment through Superdex 200 HR 10/30, and when radioactivity measurements were performed on each fraction by the method of Example 2, the activity dissociated by trypsin treatment as shown in Fig. 5 shifted to a position behind the position of elution of the original radioactivity obtained in Example 2, that is, toward the side of low-molecular weight. From the above-mentioned results, the binding site of compound A was inferred to exist in the oxygenase domain of the iNOS molecule.

### [Example 7] Construction of an expression system for human iNOS oxygenase domain (iNOSox) by E. coli

Using Human iNOS structural gene (GenBank Acc.No.X73029) cloned according to literature (Charles IG. et al. 1993, Proc. Natl. Acad. Sci. USA 90: 11419-23) as a template, amplification of genes of the oxygenase domain region (amino acids 1 to 526) by PCR was performed. After amplification using the following PCR primers containing a *Kpn*I site at the 5' side and a *Hind*III site at the 3' side, this was cloned into the same site of pTrcHis2 (Invitrogen), and iNOSox expression vector (ph-iNOSox-Trc2B) was obtained.

F1: AAAGGTACCGAGATGGCCTGTCCTTGG (SEQ ID NO: 5)
F2: AAGCTATCGAATTTGTCAAC (SEQ ID NO: 6)

F3: CACCTGACCTTGTGCTTGAG (SEQ ID NO: 7)
R1: TTTTGCCTCTTTGAAGGAGC (SEQ ID NO: 8)
R2: CCACTCGTATTTGGATGTT (SEQ ID NO: 9)
R3: CTTGAAGCTTCGCTTCTCGTCCTG (SEQ ID NO: 10)

According to the method of Ghosh et al. (Ghosh D. K. et al. 1997, Biochemistry 36, 10609-10619), transformation of a protease-deficient DE3 strain by this expression vector and its cultivation were carried out. This was followed by disruption of the collected bacterial cells, and then according to the attached protocol, the expressed protein was purified. The expressed protein was confirmed in the 200 mM imidazole eluted fraction as a protein band of 60 to 65 kDa by SDS-PAGE. This band was confirmed to be recognized specifically in Western blotting by anti-(His)6 antibody (Invitrogen), and was judged to be the desired expression protein.

### [Example 8] Restricted hydrolysis of iNOSox by trypsin

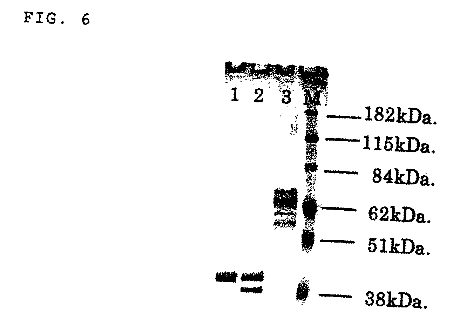

Restricted hydrolysis of human iNOSox by trypsin was performed according to the method by Ghosh et al. (Ghosh D.K.et al. 1997, Biochemistry 36, 10609-10619). Trypsin digestion (E/S = 1/10, 4°C, 1 hour) was carried out on iNOSox (100 µg/ml) purified with Ni-NTA Agarose (QIAGEN) in 25 mM Tris-HCl (pH 7.5) containing 3 ml of DTT and 2 M urea, and the reaction was stopped by adding twice the number of moles of soy bean trypsin inhibitor (STI) compared to trypsin. The result of analysis by SDS-PAGE is shown in Fig. 6. From this restricted hydrolysis, a nearly singular peak of approximately 40 kDa was obtained. When amino acid sequence analysis was performed after transferring the 40 kDa molecule band to a PVDF membrane, this was found to be a mixture of two types of molecules lacking the N-terminal amino acid residue 111 and 113.

### [Example 9] Construction, expression, and purification of Δ122-iNOSox

An expression plasmid of Δ122-iNOSox lacking the N-terminal amino acid 122 of iNOSox was constructed as a protein that mimics the 40 kDa molecule obtained in Example 8. The Δ122-iNOSox structural gene was amplified by PCR with ph-iNOSox-Trc2B (Example 7) as a template, and using the following primers containing a *Kpn*I site on the 5' side and an *Xma*I site on the 3' side. The amplified purified product was cloned into the same site of pTrcHis2 (Invitrogen) and a Δ122-iNOSox expression plasmid (pΔ122-iNOSox-Trc2B) was obtained.

Primers
F4: GGTACCGAATCTCTGGTCAAG (SEQ ID NO: 11)
R4: CCACTCGTATTTGGGATGTT (SEQ ID NO: 12)

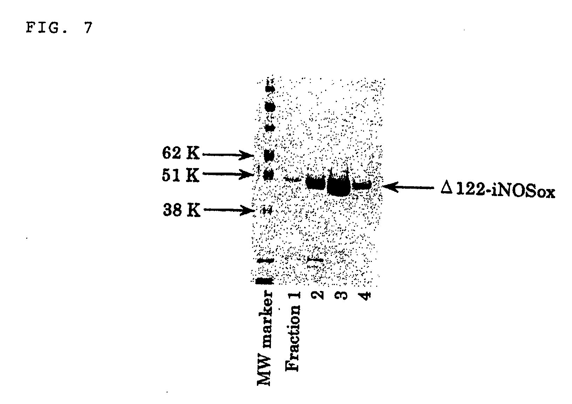

A method similar to that of Example 7, that is, the transformation of protease-deficient DE3 strain with this expression plasmid and culture was carried out. This was followed by disruption of the collected bacterial cells, and the extract containing the expressed protein was purified by Ni-NTA Agarose column chromatography. As a result, Δ122-iNOSox was eluted by concentration gradient elution with 40 mM to 200 mM imidazole and confirmed as a nearly singular band by SDS-PAGE (Fig. 7).

### [Example 10] Construction of a binding experiment system using Δ122-iNOSox

A binding assay system for ³H-compound A (25Ci/mmol) was constructed using Δ122-iNOSox obtained in Example 9. In principle, it is an assay system in which the binding strength of a compound towards iNOS can be analyzed by measuring the amount of ³H-compound A replaced by various test drugs added to bound Δ122-iNOSox protein and ³H-compound A (10 nM). That is, to 100 µL of a Δ122-iNOSox solution that has been diluted to a final concentration of 1 µg/mL with 25 mM Tris-HCl (pH 7.5) containing 150 mM NaCl and 1% CHAPS, 1 µL of 1 µM ³H-compound A was added and was incubated for 3 hours at 4°C, then the entire amount was applied to a gel filtration mini column for desalting (NICK column; Pharmacia) that was pre-equilibrated with 25 mM Tris-HCl (pH 7.5) containing 150 mM NaCl. Next, 400 µL of the same buffer was added, the eluted solution was mixed with 7 mL of aquasol, and this was measured with a liquid scintillation counter.

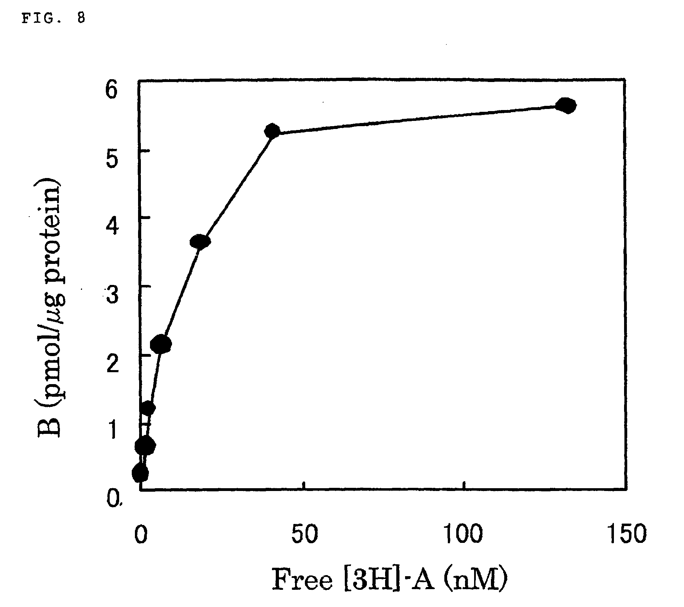

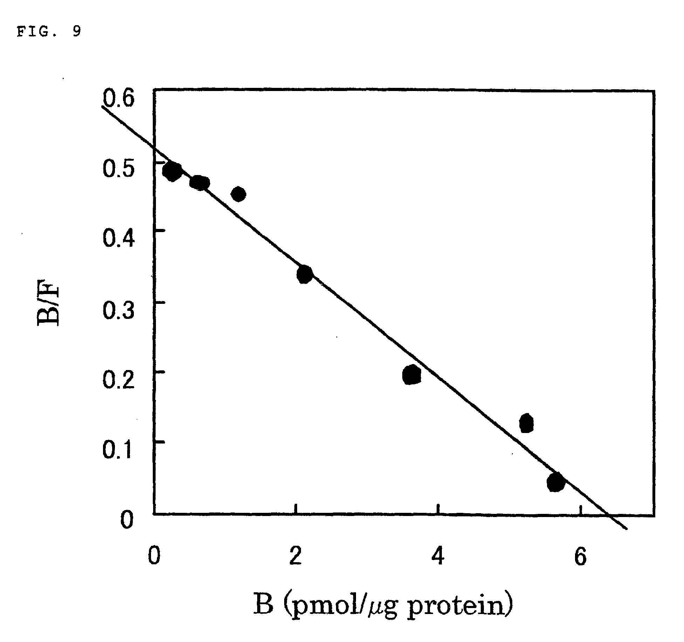

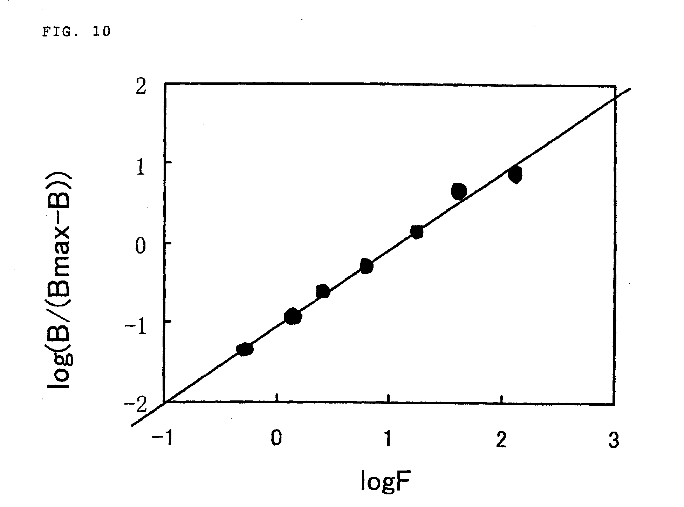

When the binding characteristics of the Δ122-iNOSox protein to ³H-compound A were investigated by this method, a Kd value of 13.5 nM was obtained from the saturation curve of Fig. 8 and its Scatchard plot (Fig. 9: correlation coefficient 0.981). Furthermore, when Hill plot (Fig. 10: correlation coefficient) analysis was performed, the Hill coefficient was 0.971, suggesting the binding of compound A to the Δ122-iNOSox protein with a 1:1 ratio.

### Industrial Applicability

This invention provides screening methods for compounds having the activity to inhibit activation of iNOS. The screening methods of this invention consist of directly detecting the effect of interfering with dimerization, which is an important step in iNOS activation. Therefore, one can expect a high specificity towards iNOS from an iNOS activation inhibitor that can be obtained by this invention.

For example, compounds that have an inhibitory effect by interfering with the enzyme reaction of NOS may act in an inhibitory manner not only towards iNOS, but also towards other enzyme reactions of isozymes. In contrast, since compounds that can be found by a screening method of this invention specifically inhibit dimerization of iNOS, high selectivity towards iNOS can be expected.

Furthermore, compounds having iNOS inhibitory activity that can be obtained by a screening method of this invention can become iNOS inhibitors having excellent rapid action properties. For example, the inhibition of iNOS can be accomplished also by interfering with its expression. However, iNOS inhibitory effect caused by the inhibition of expression, does not extend to translation of mRNA and to iNOS that already exists as a protein. In contrast, compounds that can be obtained from this invention that interfere with the dimerization of iNOS are based on the interference with dimerization, which is the final step in the process of acquiring enzyme activity of iNOS. Therefore, at the time when the agent is administered, an inhibitory effect can be expected not only towards iNOS that is in the process of being translated into a protein, but also towards iNOS that already exists as a protein. Therefore, compounds selected by a screening method of this invention provide iNOS inhibitor having excellent rapid action properties.

In addition, the screening methods of this invention can be carried out quickly with simple manipulations. That is, screening can be performed by a binding assay that uses as an index, the binding of a candidate compound towards a monomer (or a mutant thereof) constituting iNOS. Compared to methods that include culturing steps, results can be obtained in a short time with a screening that uses a binding reaction as an index. Therefore, an efficient screening can be carried out utilizing the quickness of HTS. In contrast, known screening methods that use, for example, the iNOS expression inhibitory effect as an index, are based on reporter assays using the 5' flanking region of the iNOS gene. In a reporter assay system, the construction of transformants and their culturing process are necessary. Such manipulations require a long time and also expertise to perform highly reproducible experiments.

As mentioned above, the screening methods according to this invention allow quick screening of iNOS inhibitory compounds having excellent specificity and rapid action properties with simple manipulations.

## Claims

1. A method of *in vitro* screening for a compound that inhibits dimer formation of inducible nitrogen oxide synthase, wherein the method comprises the steps of:
a) contacting candidate compounds with an inducible nitrogen oxide synthase monomer, or a mutant thereof,
b) selecting a compound that binds to the inducible nitrogen oxide synthase monomer, or the mutant thereof.

2. The method of claim 1, wherein the mutant of the inducible nitrogen oxide synthase monomer is a protein containing an amino acid sequence in which at least one amino acid selected from cysteine at position 110, lysine at position 113, and cysteine at position 115 has been deleted or mutated.

3. The method of claim 2, wherein the cysteine at position 110 of the protein has been deleted or mutated.

4. The method of claim 2, wherein the mutant of the inducible nitrogen oxide synthase monomer has amino acid residues up to position 122 on the N-terminal side in its oxygenase domain deleted or mutated.

5. The method of any one of claims 2 to 4, wherein the mutant of the inducible nitrogen oxide synthase monomer is a protein that lacks the reductase domain.

6. The method of claim 1, wherein the binding of the a candidate compound to the oxygenase domain monomer of inducible nitrogen oxide synthase, or to a mutant thereof, is detected by competitive inhibition of a binding ligand towards the oxygenase domain monomer, or the mutant thereof, by the candidate compound.

7. The method of claim 6, wherein the ligand for competitive inhibition is a compound that inhibits dimerization of monomers by binding to the oxygenase domain of inducible nitrogen oxide synthase.

8. The method of claim 7, wherein the ligand is a compound that does not bind to heme.

9. An inhibitor of inducible nitrogen oxide synthase activation, wherein the inhibitor comprises as a main ingredient a compound that can be obtained by any one of the methods of claims 1 to 8.

10. An agent for treating a disease selected from the group consisting of graft rejection following an organ transplant, cerebral infarction, and cardiovascular ischemia, wherein the agent comprises the inhibitor of claim 9.

11. A protein according to any one of (a) to (c) below:
(a) a protein comprising the amino acid sequence of SEQ ID NO: 4,
(b) a monomeric stable protein having the activity to bind to a compound that inhibits dimerization by binding to inducible nitrogen oxide synthase, wherein the protein comprises an amino acid sequence wherein one or several amino acids in the amino acid sequence of SEQ ID NO: 4 has been replaced, deleted, inserted, and/or added, and
(c) a monomeric stable protein having the activity to bind to a compound that inhibits dimerization by binding to inducible nitrogen oxide synthase, which is encoded by DNA that hybridizes under stringent conditions to DNA comprising the nucleotide sequence of SEQ ID NO: 3.

12. A DNA encoding any one of the proteins of claim 11.

13. An expression vector containing the DNA of claim 12.

14. A transformant that harbors the DNA of claim 12 or the expression vector of claim 13 in an expressive manner.

15. A method for producing the protein of claim 11, comprising cultivating the transformant of claim 14, and collecting an expression product from the transformant and/or its culture supernatant.

16. A method for detecting the activity of a candidate compound to inhibit dimer formation of inducible nitrogen oxide synthase comprising the steps of:
a) contacting the candidate compound with the protein of claim 11, and
b) detecting the binding of the candidate compound to the protein.

17. A method of screening for a compound that inhibits dimer formation of inducible nitrogen oxide synthase, comprising the step of selecting the candidate compound in which binding to the protein was detected in the method of claim 16.
